# EUROPEAN PATENT SPECIFICATION

(11) **EP 3 834 788 B1**
(45) Date of publication and mention of the grant of the patent: **17.01.2024**
(21) Application number: 19848029.5
(22) Date of filing: 08.08.2019
(51) Int. Cl.: A61F 13/47, A61F 13/475, A61F 13/53, A61F 13/49

(54) **ADULT DISPOSABLE DIAPER**
WEGWERFWINDEL FÜR ERWACHSENE
COUCHE JETABLE POUR ADULTE

(30) Priority: 10.08.2018 JP 2018152104
(43) Date of publication of application: 16.06.2021
(73) Proprietor: Koyo Corporation, Yokohama-shi, Kanagawa 236-0004 (JP)
(72) Inventor: SHIRAI, Atsuko, Yokohama-shi Kanagawa 236-0004 (JP)
(74) Representative: Samson & Partner Patentanwälte mbB
(86) International application number: PCT/JP2019/031512
(87) International publication number: WO 2020/032217

(56) References cited:
- WO-A1-01/45622
- JP-A- H1 199 176
- JP-A- 2008 161 251
- JP-B2- 5 982 272
- US-A1- 2014 031 783
- US-A1- 2016 235 602
- US-B2- 9 320 658

## Description

### TECHNICAL FIELD

The present invention relates to adult disposable diapers.

### BACKGROUND ART

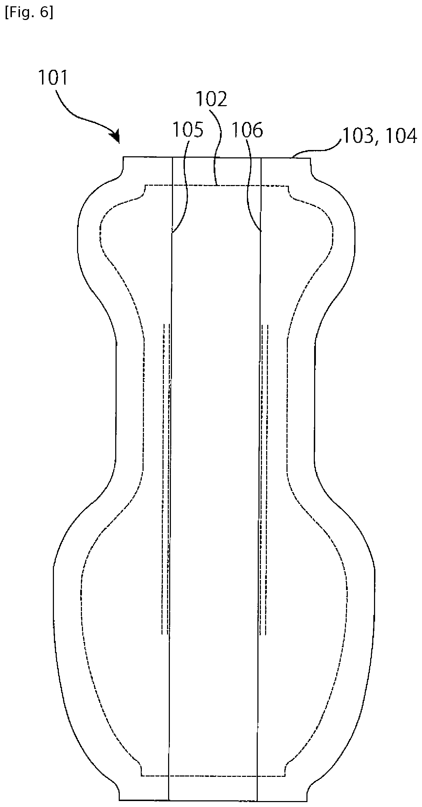

As shown in FIG. 6, the diaper 101, which is known as a disposable diaper for adults, includes a gourd-shaped absorbent core 102 with a narrowed middle part in the longitudinal direction, a liquid-permeable front sheet 103 and a liquid-impermeable back sheet 104 with the absorbent core 102 sandwiched between them, left and right rising gathered units 105 and 106 extending in the longitudinal direction on the surface sheet 103.

The diaper 101 has a narrow middle portion corresponding to the wearer's crotch, and the absorbent core 102 located in the middle portion is small. Thus, if urine is excreted while a wearer is lying sideways, the urine may not be sufficiently absorbed by the absorbent core 102 in the middle portion and may leak to the outside.

Patent Document 1 discloses a disposable diaper that includes a rectangular absorbent core, a rectangular front sheet and back sheet with the absorbent core sandwiched between them, left and right three-dimensional leak-proof structure forming sheets extending in the longitudinal direction on the surface sheet. The front region and the rear region of the absorbent core are provided with notches that penetrate inward in the width direction and inward in the longitudinal direction from the left and right side edges thereof. When the diaper is worn, the absorbent core is deformed into a boat shape by bending the portion between the front and rear notches toward the surface sheet side. Further, free ends of the left and right leak-proof structure forming sheets stand up.

The diaper has a low fit with the wearer because the absorbent core deforms into the boat shape. Thus, if urine is excreated while the wearer is lying sideways, the urine may leak to the outside through a gap formed between the three-dimensional leak-proof structure forming sheet and the wearer.

### RELATED ART DOCUMENTS

### PATENT DOCUMENTS

Patent document 1: Japanese Patent No. 5982272

### SUMMARY OF THE INVENTION

### PROBLEMS TO BE SOLVED BY THE INVENTION

The present invention has an object to provide an adult disposable diaper that is excellent in preventing liquid leakage when the wearer is lying sideways. The present invention has another object to provide an adult disposable diaper having an excellent fit to the wearer.

### MEANS OF SOLVING THE PROBLEMS

According to one aspect of the present invention, an adult disposable diaper includes an absorber, a laminated sheet in which a plurality of sheets are laminated so as to envelope the absorber, and left and right rising gathered units to extend in a longitudinal direction on a wearer-facing surface of the laminated sheet, wherein the diaper is divided into a front section, a middle section and a back section corresponding to a lower abdomen, a crotch and buttocks of a wearer, respectively, wherein the absorber includes left and right first notches to extend inward in a width direction of the absorber in a boundary region between the front section and the middle section and includes left and right second notches to extend inward in the width direction of the absorber in a boundary region between the middle section and the back section, wherein the laminated sheet includes left and right third notches to extend inward in a width direction of the laminated sheet toward the first notches in the boundary region between the front section and the middle section, wherein left and right thigh absorbers that are used by being bent so as to face inner thighs in the crotch of the wearer are formed by left and right side parts of the absorber in the middle section, and wherein elastic bodies are provided at roots of the left and right thigh absorbers in a region opposite to the wearer-facing surface in the laminated sheet.

According to another aspect of the present invention, the laminated sheet includes left and right fourth notches to extend inward in the width direction of the laminated sheet toward the second notches in the boundary region between the middle section and the back section. According to still another aspect of the present invention, the adult disposable diaper includes a leak-proof structure that covers from a tip to a root of both a wearer-facing surface and an opposite surface thereof in the left and right thigh absorbers.

The present invention is not limited to the above aspect. Further details, features, functions, and/or effects of the present invention will be more apparent from the following description of preferred exemplary embodiments of the present invention.

### BRIEF DESCRIPTION OF THE DRAWINGS

FIG. 1 is a perspective view of an adult disposable diaper, which is an embodiment of the present invention.
FIG. 2 is a plan view showing an adult disposable diaper according to an embodiment of the present invention in a state where an elastic body does not exert a contraction force.
FIG. 3 is a cross-sectional view of an adult disposable diaper in a worn state taken along line III-III in FIG. 2.
FIG. 4 is a plan view showing an adult disposable diaper according to another embodiment of the present invention in a state where an elastic body does not exert a contraction force.
FIG. 5 is a cross-sectional view of an adult disposable diaper in a worn state taken along line V-V in FIG. 4.
FIG. 6 is a plan view showing a conventional adult disposable diaper in a state where an elastic body does not exert a contraction force.

### MODE FOR CARRYING OUT THE INVENTION

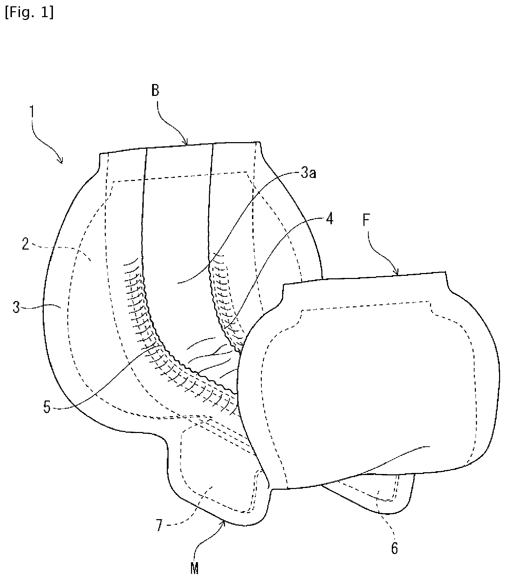

FIGs. 1 to 3 show an adult disposable diaper 1 which is an embodiment of the present invention. The diaper 1 is attached to an inner surface of a pants-type disposable diaper or wear (not shown) and is used as a urine absorbing pad, an incontinence pad, or the like. As shown in FIG. 1, the diaper 1 includes an absorber 2, a laminated sheet 3 in which a plurality of sheets are laminated so as to envelope the absorber 2, left and right rising gathered units 4 and 5 to extend in a longitudinal direction on a wearer-facing surface 3a of the laminated sheet 3, and left and right thigh absorbers 6 and 7 that are used by being bent so as to face inner thighs in a crotch of a wearer

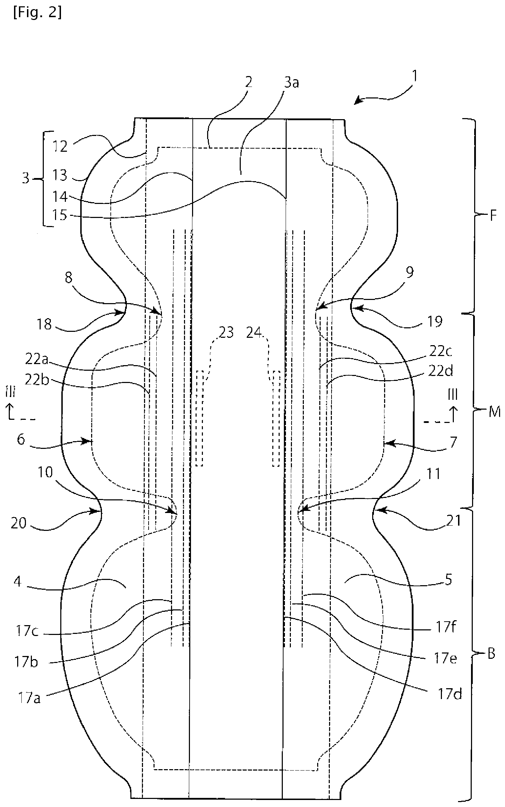

As shown in FIGs. 1 and 2, the diaper 1 is divided into a front section F, a middle section M, and a back section B corresponding to a lower abdomen, a crotch, and buttocks of a wearer, respectively. The diaper 1 has a longitudinal direction as a front-back direction and a width direction as a left-right direction.

The absorber 2 has a liquid retention property, and quickly absorbs a liquid such as urine discharged from the wearer and retains it inside. The raw material of the absorber 2 includes, for example, a water-absorbent fiber obtained by defibrating a roll sheet-like pulp and a powdery or granular superabsorbent polymer. These raw materials are air-conveyed to a molding die installed on the peripheral surface of the suction drum, and are deposited and molded in the molding die while being sucked. An absorber is manufactured by sandwiching or wrapping such a molded product with water-absorbent paper or non-woven fabric (not shown).

In a boundary region between the front section F and the middle section M, a left first notch 8 and a right first notch 9 to extend inward in a width direction are provided from each of the left and right side edges of the absorber 2. In a boundary region between the middle section M and the back section B, a left second notch 10 and a right second notch 11 to extend inward in the width direction are provided from each of the left and right side edges of the absorber 2. The distance between the left and right first notches 8 and 9 is larger than the distance between the left and right second notches 10 and 11, but is not limited to this, and may be equal or smaller.

The maximum width in the left-right direction of the absorber 2 in the middle section M is larger than the maximum width in the left-right direction of the absorber 2 in the front section F, but is not limited to this, and may be equal or smaller. The maximum width in the left-right direction of the absorber 2 in the middle section M is equal to the maximum width in the left-right direction of the absorber 2 in the back section B, but is not limited to this, and may be small or large. The absorber 2 in the middle section M can form the left and right thigh absorbers 6 and 7 which are used by being bent so as to face the inner thigh of the wearer's crotch. The absorber 2 in the middle section M can be of various sizes and shapes suitable for the wearer of the adult diaper 1 to sufficiently absorb and retain the urine excreted while lying sideways.

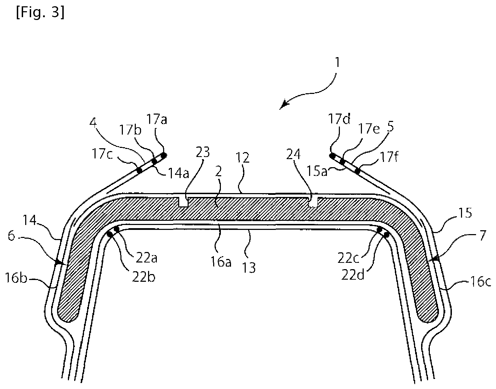

As shown in FIGs. 2 or 3, the laminated sheet 3 includes a top sheet 12, a bottom sheet 13, a left gather sheet 14, a right gather sheet 15, and liquid impermeable sheets 16a, 16b and 16c. In FIG. 2, the liquid impermeable sheets 16a, 16b are 16c are omitted. The length in the front-back direction of each sheet constituting the laminated sheet 3 is the same and is longer than the length in the front-back direction of the absorber 2.

The top sheet 12 has a rectangular shape. The top sheet 12 is arranged on the upper surface of the absorber 2, that is, the wearer-facing surface, so as to cover the central portion excluding the left and right side portions of the absorber 2. The width in the left-right direction of the top sheet 12 is smaller than the maximum width of each of the front section F, the middle section M, and the back section B of the absorber 2. The top sheet 2 covers the inner portions of the left and right first notches 8 and 9 and the inner portions of the left and right second notches 10 and 11. The top sheet 12 has liquid permeability and allows liquid such as urine to permeate the top sheet 12 and move into the absorber 2.

The bottom sheet 13 is an exterior sheet of the diaper 1. The bottom sheet 13 is arranged under the lower surface of the absorber 2, that is, the surface opposite to the wearer facing surface, so as to cover the entire absorber 2. In any of the front section F, the middle section M and the back section B, the width in the left-right direction of the bottom sheet 13 is larger than the width in the left-right direction of the absorber 2. A liquid-impermeable sheet 16a having substantially the same shape as the bottom sheet 13 is arranged between the bottom sheet 13 and the absorber 2 and is joined with an adhesive or the like. The bottom sheet 13 and the liquid-impermeable sheet 16a prevent the liquid that has permeated the top sheet 3 and the liquid that has not been absorbed or held by the absorber 2 from leaking to the outside. Since the bottom sheet 13 made of non-woven fabric covers the outside of the liquid-impermeable sheet 16a made of polyethylene or polyester film, the diaper 1 is hard to slip and feels good on the skin when it is attached to an outer diaper or underwear.

The left gather sheet 14 is arranged on the upper surface of the absorber 2, that is, the wearer-facing surface, so as to cover the left side portion of the absorber 2. The left edge of the left gather sheet 14 has substantially the same contour as the left edge of the bottom sheet 13. The right side portion of the left gather sheet 14 has a folded-back portion 14a toward the top sheet 12 with the right side edge as a crease.

The liquid-impermeable sheet 16b is provided between the left side portion of the left gather sheet 14 and the left side portion of the absorber 2, and is bonded to them with an adhesive or the like. The left edge of the liquid-impermeable sheet 16b has substantially the same contour as the left edge of the bottom sheet 13.

Elastic bodies 17a, 17b and 17c are provided in parallel between the left gather sheet 14 and its folded-back portion 14a in order from the crease, and extend in the front-back direction except for both ends thereof. The left gather sheet 14 including the folded-back portion 14a is fixed to another sheet of the laminated sheet 3 with an adhesive or the like at the left side portion and both ends of the front-back direction. The left gather sheet 14 is gathered by the contraction of the elastic bodies 17a, 17b and 17c, and the right side portion of the left gather sheet 14 stands up from the upper surface of the top sheet 12. In this way, the left rising gathered unit 4 extending in the longitudinal direction is formed on the upper surface of the top sheet 12 which is the wearer-facing surface 3a of the laminated sheet 3.

The right gather sheet 15 is provided on the upper surface of the absorber 2, that is, the wearer-facing surface, so as to cover the right side portion of the absorber 2. The right edge of the right gather sheet 15 has substantially the same contour as the right edge of the bottom sheet 13. The left side portion of the right gather sheet 15 has a folded-back portion 15a toward the top sheet 12 with the left side edge as a crease.

The liquid-impermeable sheet 16c is provided between the right side portion of the right gather sheet 15 and the right side portion of the absorber 2, and is joined to them with an adhesive or the like. The right edge of the liquid-impermeable sheet 16c has substantially the same contour as the right edge of the bottom sheet 13.

Elastic bodies 17d, 17e and 17f are provided in parallel between the right gather sheet 15 and its folded-back portion 15a in order from the crease, and extend in the front-back direction except for both ends. The right gather sheet 15 including the folded-back portion 15a is fixed to another sheet of the laminated sheet 3 with an adhesive or the like at the right side portion and both ends of the front-back direction. The right gather sheet 15 is gathered by the contraction of the elastic bodies 17d, 17e and 17f, and the left side portion of the right gather sheet 15 stands up from the upper surface of the top sheet 12. In this way, the right rising gathered unit 5 extending in the longitudinal direction is formed on the upper surface of the top sheet 12 which is the wearer-facing surface 3a of the laminated sheet 3.

As a raw material for the top sheet 12, for example, a liquid-permeable non-woven fabric made of chemical fibers such as polyethylene, polypropylene, polyester, nylon and rayon, natural fibers such as cotton and cellulose, or a combination thereof can be used. The top sheet 12 is not limited to a non-woven fabric, and may be, for example, a perforated film or tissue formed so as to be liquid permeable, or a laminate thereof, or a sheet formed by appropriately connecting them.

The bottom sheet 13, the left gather sheet 14, and the right gather sheet 15 have liquid impermeability or water repellency, and are, for example, non-woven fabrics made of chemical fibers such as polyethylene, polypropylene, polyester, nylon and rayon, natural fibers such as cotton and cellulose, or raw materials such as combinations thereof. The bottom sheet 13, the left gather sheet 14, and the right gather sheet 15 are not limited to non-woven fabrics, and may be, for example, resin films, tissues, elastic materials, or laminates thereof, or sheets formed by appropriately connecting them.

The liquid-impermeable sheets 16a, 16b and 16c are made of a liquid-impermeable material such as a film such as polyethylene, polypropylene, polyester, or nylon, or a coated non-woven fabric.

The laminated sheet 3 and each sheet constituting the laminated sheet 3 are not limited to those described above, and can adopt various sizes, shapes, and lamination orders suitable for enveloping the absorber 2 and allowing the absorber 2 to absorb and retain the liquid such as urine excreted from the wearer without leaking to the outside.

The elastic bodies 17a, 17b, 17c, 17d, 17e, and 17f are made of, for example, stretchable thread-like raw materials such as natural rubber, polyurethane resin, and stretchable hot melt. These elastic bodies are not limited to the thread shape, but may be strip-shaped or sheet-shaped.

In the boundary region between the front section F and the middle section M, a left third notch 18 extending inward in the width direction of the laminated sheet 3 is provided from the left edge of the laminated sheet 3 toward the left first notch 8. Further, a right third notch 19 extending inward in the width direction of the laminated sheet 3 is provided from the right edge of the laminated sheet 3 toward the right first notch 9.

In the boundary region between the middle section M and the back section B, a left fourth notch 20 extending inward in the width direction of the laminated sheet 3 is provided from the left edge of the laminated sheet 3 toward the left second notch 10. Further, a right fourth notch 21 extending inward in the width direction of the laminated sheet 3 is provided from the right edge of the laminated sheet 3 toward the right second notch 11. The distance between the left and right third notches 18 and 19 is smaller than the distance between the left and right fourth notches 20 and 21, but the distance is not limited to this and may be equal or larger.

The maximum width in the left-right direction of the laminated sheet 3 in the middle section M is larger than the maximum width in the left-right direction of the laminated sheet 3 in the front section F, and is equal to the maximum width in the left-right direction of the laminated sheet 3 in the back section B. The maximum width in the left-right direction of the laminated sheet 3 in the middle section M is larger than the maximum width in the left-right direction of the laminated sheet 3 in the front section F, and is equal to the maximum width in the left-right direction of the laminated sheet 3 in the back section B.

The left side part of the absorber 2 in the middle section M is located between the left first notch 8 and the left second notch 10, and forms the left thigh absorber 6 to be used by being bent so as to face the inner thigh of the wearer's crotch. The right side part of the absorber 2 in the middle section M is located between the right second notch 9 and the right second notch 11, and forms the right thigh absorber 7 to be used by being bent so as to face the inner thigh of the wearer's crotch. The left and right thigh absorbers 6 and 7 greatly project in the left and right sides of the absorber 2 in the middle section M.

The left and right thigh absorbers 6 and 7 of the absorber 2 in the middle section M are located between the first notches 8 and 9 and the second notches 10 and 11, and can be bent at the roots of the thigh absorbers 6 and 7. Further, the laminated sheet 3 around the thigh absorbers 6 and 7 has third notches 18 and 19 and fourth notches 20 and 21. Thus, the thigh absorbers 6 and 7 of the absorber 2 can be bent and used so as to face the inner thigh of the wearer's crotch. The shapes and sizes of the third notches 18 and 19 and the fourth notches 20 and 21 formed in the laminated sheet 3 are not limited to the shapes and sizes as shown in FIG. 2. The thigh absorbers 6 and 7 may be of various shapes and sizes suitable for use by bending them so as to face the inner thigh of the wearer's crotch.

The fourth notch may be omitted as long as the thigh absorber of the absorber can be bent and used so as to face the inner thigh of the wearer's crotch.

As shown in FIG. 1, the thigh absorbers 6 and 7 project greatly to the left and right sides of the absorber 2 in the middle section M of the diaper 1, and can be bent and arranged so as to face the inner thigh of the wearer's crotch. Thus, the front section F, the middle section M, and the back section B of the diaper 1 can be arranged close to or in contact with the lower abdomen, the crotch, and the buttocks of the wearer, respectively, so that the diaper 1 has a good fit to the wearer and high leakage prevention ability. Further, even when the wearer of the diaper 1 is sleeping sideways, since the thigh absorbers 6 and 7 are large, the ability to absorb and retain the excreted urine and the like and prevent leakage is high. Even if the diaper 1 has a high fit to the wearer, the left and right rising gathered units 4 and 5 may stand up on the top sheet 12 to a position where they function as a three-dimensional leakage-proof structure.

As shown in FIGs. 2 or 3, elastic bodies 22a and 22b are provided at the roots of the left thigh absorber 6, that is, the region connecting the most inner portion of the left first notch 8 and the most inner portion of the left second notch 10 in the absorber 2. Elastic bodies 22c and 22d are provided at the roots of the right thigh absorber 7, that is, the region connecting the most inner portion of the right first notch 9 and the most inner portion of the right second notch 11 in the absorber 2. The elastic bodies 22a, 22b, 22c, and 22d are fixed to the bottom sheet 13 with an adhesive or the like under the absorber 2.

The elastic bodies 22a, 22b, 22c, 22d are made of stretchable thread-like raw materials such as natural rubber, polyurethane resin, and stretchable hot melt. These elastic bodies are not limited to thread-shaped elastic bodies, but may be strip-shaped or sheet-shaped elastic bodies.

The elastic bodies 22a, 22b, 22c, and 22d are a kind of guide units for bending the left and right side parts of the absorber 2 in the middle section M so that the thigh absorbers 6 and 7 face the inner thigh of the wearer's crotch. By contracting the elastic bodies 22a, 22b, 22c, and 22d provided at the roots of the thigh absorbers 6 and 7, the thigh absorbers 6 and 7 can be easily bent at and around the roots thereof and can easily face the inner thigh of the wearer's crotch.

The guide unit prevents bending in an undesired direction or position, such as the left and right side portions of the absorber 2 in the middle section M being bent in an oblique direction or in an asymmetrical position. This also improves leak resistance. The position where the guide unit is provided may be a position that extends in the front-back direction between the crotch and the thigh of the wearer when the diaper is worn. It is desirable that one or more guide units are provided on each of the left and right sides. Providing many guide units makes it more reliable to keep the diaper in the correct position of the wearer. This is because the bending angles and positions of the absorber 2 and the thigh absorbers 6 and 7 in the middle section M change between the crotch and the thigh depending on the movement, body shape and posture of the wearer.

Since the diaper 1 has large thigh absorbers 6 and 7 on the left and right sides in the middle section M, the width of the absorber 2 between the left and right thigh absorbers 6 and 7 in the middle section M can be narrowed to improve the fit to the crotch portion of the wearer. For example, in the conventional diaper 101 shown in FIG. 6, the width of the central portion of the absorber 102 arranged to face the crotch portion of the wearer is usually 100 mm for the small size for adults, 130 mm for the middle size, and 160 mm for the large size. On the other hand, in the diaper 1, the width of the central portion of the absorber 2 arranged to face the crotch portion of the wearer is 80 mm for the small size for adults, 100 mm for the middle size, and 120 mm for the large size. The diaper 1 provided with the large left and right thigh absorbers 6 and 7 has a width of the central portion of the absorber 2 arranged to face the crotch portion of the wearer, which is 20 mm to 40 mm narrower than that of the conventional diaper 101. Thus, the diaper 1 improves the fit to the wearer's crotch.

As shown in FIGs. 2 and 3, the diaper 1 is provided with left and right recessed grooves 23 and 24 extending in the front-back direction in or near a region where the inner edges of the left and right gather sheets 14 and 15 are projected onto the middle area in the width direction of the absorber 2. The left and right grooves 23 and 24 are a kind of guide units for bending the thigh absorber 6 and 7. The absorber 2 can be easily bent at the positions of the grooves 23 and 24 closer to the center than the roots of the thigh absorbers 6 and 7. Thus, the diaper 1 has a higher fit to the crotch of the wearer, and the diaper 1 faces the inner thigh of the crotch of the wearer to further improve the fit.

Liquid permeable sheets 16b and 16c are provided between the wearer-facing surfaces of the left and right thigh absorbers 6 and 7 and the left and right gather sheets 14 and 15, respectively. A liquid-impermeable sheet 16a is provided between the opposite surfaces of the wearer-facing surfaces of the left and right thigh absorbers 6 and 7 and the bottom sheet 13. As shown in FIG. 3, a leak-proof structure is provided so as to cover from the tip to the root of the thigh absorbers 6 and 7 on both the wearer-facing surface and the opposite surface. Thus, whether the wearer of the diaper 1 is standing, sitting, lying on his back, lying down, or lying sideways, it is possible to prevent liquids such as urine absorbed by the thigh absorbers 6 and 7 from leaking to the outside.

A comparative experiment of absorption capacity was carried out for Example 1 of the present invention having the shape and structure of the diaper 1 shown in FIG. 2 and the conventional example having the shape and structure of the diaper 101 shown in FIG. 6. In both the first embodiment and the conventional example, the length of the laminated sheet in the front-back direction is 560 mm and the maximum width is 260 mm, and the length of the absorber in the front-back direction is 515 mm and the maximum width is 210 mm. In the absorber 2 of Example 1, the total pulp amount is 34.4 g, the total SAP amount is 12.1 g, and the area of the wearer-facing surface is 0.099 m². In the absorber 102 of the conventional example, the total amount of pulp is 32.0 g, the total amount of SAP is 11.0 g, and the area of the wearer-facing surface is 0.092 m². The total pulp amount, the total SAP amount, and the area of the wearer-facing surface of the absorber 2 of Example 1 are 1.1 times or less of those of the absorber 102 of the conventional example, and are substantially equal. In the first embodiment and the conventional example, the thickness of the absorber is also the same.

For the Example 1 and the conventional example, a comparative experiment of absorption capacity was carried out when the wearer lay on his back and when he lay on his side. Specifically, each diaper was attached to a doll conforming to an adult standard body shape, and artificial urine was injected at 15 ml / sec until it leaked beyond the rising gathered portions. The amount of absorption was calculated by subtracting the weight of the diaper before absorption from the weight of the diaper after absorption. This experiment was carried out 5 times each, and the average absorption amounts of Example 1 and the conventional example were obtained and compared.

According to the results of the above-mentioned experiment, when the wearer lay on his back, the average absorption amount of Example 1 was 797 g, which was 1.4 times or more larger than that of the conventional example of 561 g. When the wearer lay down sideways, the average absorption amount of Example 1 was 542 g, which was 1.3 times larger than that of the conventional example of 424 g. In this way, according to the diaper 1 of the present invention, the left and right large thigh absorbers 6 and 7 in the middle section M are bent so as to face each other along the inner thigh of the wearer's crotch, and thus, it was confirmed that the leakage of urine and the like can be effectively prevented regardless of whether the wearer sleeps on his back or sideways.

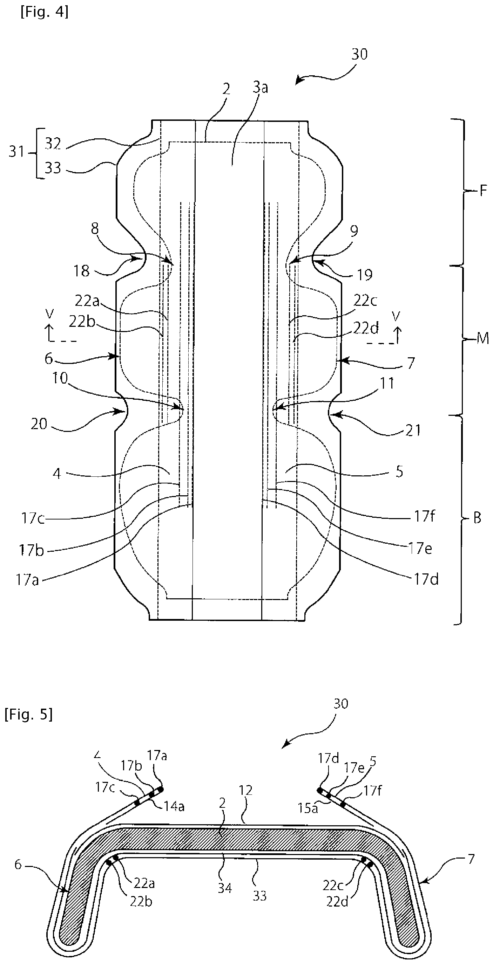

Next, FIGs. 4 and 5 show an adult disposable diaper 30 which is another embodiment of the present invention. The elements of the diaper 30 which are the same as the elements of the diaper 1 are designated by the same reference numerals, and the description thereof will be omitted as appropriate.

As shown in FIGs. 4 and 5, the diaper 30 does not have grooves 23 and 24 formed in the middle area of the width direction of the absorber 2. A laminated sheet 31 of the diaper 30 includes a top sheet 32, a bottom sheet 33, and a liquid-impermeable sheet 34. In FIG. 4, the liquid impermeable sheet 14 is omitted. A top sheet 32 is the same as the top sheet 12 of the diaper 1. A bottom sheet 33 has a structure in which the bottom sheet 13, the left gather sheet 14, and the right gather sheet 15 of the diaper 1 are continuously integrated and have these functions. A liquid-impermeable sheet 34 has a structure in which the liquid-impermeable sheets 16a, 16b, and 16c of the diaper 1 are continuously integrated and has a function thereof. Since the bottom sheet 33 and the liquid-impermeable sheet 34 have an integral structure as described above, there are few steps for adhering each sheet in the diaper 30.

As shown in FIG. 4, in the front section F, the middle section M, and the back section B of the diaper 30, the maximum widths in the left-right direction of the laminated sheets 31 are equal to each other and slightly larger than the maximum widths in the left-right direction of the absorber 2. The left and right side edges of the laminated sheet 31 extend in the front-back direction in parallel at a position slightly outside the maximum width in the left-right direction of the absorber 2. In the boundary region between the front section F and the middle section M, the absorber 2 is provided with the left and right first notches 8 and 9, and the laminated sheet 31 is provided with the left and right third notches 18 and 19. In the boundary region between the middle section M and the back section B, the absorber 2 is provided with the left and right second notches 10 and 11, and the laminated sheet 31 is provided with the left and right fourth notches 20 and 21. Since the maximum width in the left-right direction of the laminated sheet 31 is slightly wider than the maximum width in the left-right direction of the absorber 2, the left-right width of the diaper 30 can be slimmed down, and the raw material of the laminated sheet 3 can be reduced.

The present invention is not limited to each of the above embodiments. The scope of the present invention includes at least a modification that is obvious from each of the above embodiments and a modification that does not change the essence of the present invention.

### INDUSTRIAL APPLICABILITY

The adult disposable diaper of the present invention can be used alone as a diaper, or can be attached to the inner surface of a disposable diaper or a wearable object and used as a urine absorbing pad, an incontinence pad, or the like. In particular, the adult disposable diapers of the present invention are suitable for wearers who often lie down.

### DESCRIPTION OF THE REFERENCE NUMERAL

1,30: adult disposable diaper,
2: absorber,
3,31: laminated sheet,
3a: wearer-facing surface,
4,5: rising gathered unit,
6,7: thigh absorber
8,9: first notch,
10,11: second notch,
12,32: top sheet,
13,33: bottom sheet,
14,15: gather sheet,
16a, 16b, 16c, 34: impermeable sheet,
17a, 17b, 17c, 17d, 17e, 17f: elastic body,
18, 19: third notch,
20, 21: fourth notch,
22a, 22b, 22c, 22d: elastic body,
23, 24: groove,
F: front section,
M: middle section,
B: back section

## Claims

1. An adult disposable diaper (1) comprising:
an absorber (2);
a laminated sheet (3) in which a plurality of sheets are laminated so as to envelope the absorber (2); and
left and right rising gathered units (4, 5) to extend in a longitudinal direction on a wearer-facing surface (3a) of the laminated sheet (3),
wherein the diaper (1) is divided into a front section (F), a middle section (M) and a back section (B) corresponding to a lower abdomen, a crotch and buttocks of a wearer, respectively,
wherein the absorber (2) comprises left and right first notches (8, 9) to extend inward in a width direction of the absorber (2) in a boundary region between the front section (F) and the middle section (M) and comprises left and right second notches (10, 11) to extend inward in the width direction of the absorber (2) in a boundary region between the middle section (M) and the back section (B),
wherein the laminated sheet (3) comprises left and right third notches (18, 19) to extend inward in a width direction of the laminated sheet (3) toward the first notches (8, 9) in the boundary region between the front section (F) and the middle section (M),
wherein left and right thigh absorbers (6, 7) that are used by being bent so as to face inner thighs in the crotch of the wearer are formed by left and right side parts of the absorber (2) in the middle section (M),
wherein elastic bodies (22a, 22b, 22c, 22d) are provided at roots of the left and right thigh absorbers (6, 7) in a region opposite to the wearer-facing surface (3a) in the laminated sheet (3), and
wherein left and right recessed grooves (23, 24) to extend in the front-back direction are provided closer to the center of the absorber (2) than the roots of the left and right thigh absorbers (6,7).

2. The adult disposable diaper (1) according to claim 1,
wherein the laminated sheet (3) further comprises left and right fourth notches (20, 21) to extend inward in the width direction of the laminated sheet (3) toward the second notches (10, 11) in the boundary region between the middle section (M) and the back section (B).

3. The adult disposable diaper (1) according to claim 1,
further comprising a leak-proof structure (16a, 16b, 16c) that covers from a tip to a root of both a wearer-facing surface and an opposite surface thereof in the left and right thigh absorbers (6, 7).

## Patentansprüche

1. Wegwerfwindel für Erwachsene (1), umfassend:
einen Absorber (2);
eine laminierte Schicht (3), bei der eine Vielzahl von Schichten so laminiert sind, dass sie den Absorber (2) umhüllen; und
linke und rechte aufsteigende geraffte Einheiten (4, 5), die sich in einer Längsrichtung auf einer dem Träger zugewandten Oberfläche (3a) der laminierten Schicht (3) erstrecken,
wobei die Windel (1) in einen vorderen Abschnitt (F), einen mittleren Abschnitt (M) und einen hinteren Abschnitt (B) unterteilt ist, die jeweils zu einem Unterbauch, einem Schritt und einem Gesäß eines Trägers korrespondieren,
wobei der Absorber (2) linke und rechte erste Kerben (8, 9) umfasst, um sich in einer Breitenrichtung des Absorbers (2) in einem Grenzbereich zwischen dem vorderen Abschnitt (F) und dem mittleren Abschnitt (M) nach innen zu erstrecken, und linke und rechte zweite Kerben (10, 11) umfasst, um sich in einem Grenzbereich zwischen dem mittleren Abschnitt (M) und dem hinteren Abschnitt (B) in der Breitenrichtung des Absorbers (2) nach innen zu erstrecken,
wobei die laminierte Schicht (3) linke und rechte dritte Kerben (18, 19) umfasst, die sich in einer Breitenrichtung der laminierten Schicht (3) nach innen in Richtung der ersten Kerben (8, 9) in dem Grenzbereich zwischen dem vorderen Abschnitt (F) und dem mittleren Abschnitt (M) erstrecken,
wobei linke und rechte Schenkelabsorber (6, 7), die verwendet werden, indem sie so gebogen werden, dass sie den inneren Schenkeln im Schritt des Trägers zugewandt sind, durch linke und rechte Seitenteile des Absorbers (2) im mittleren Abschnitt (M) gebildet werden,
wobei elastische Körper (22a, 22b, 22c, 22d) an den Fußpunkten des linken und rechten Schenkelabsorbers (6, 7) in einem Bereich gegenüber der dem Träger zugewandten Oberfläche (3a) in der laminierten Schicht (3) bereitgestellt sind, und
wobei linke und rechte vertiefte Kerben (23, 24), die sich in der Vorwärts-RückwärtsRichtung erstrecken, näher an der Mitte des Absorbers (2) bereitgestellt sind als die Fußpunkte der linken und rechten Schenkelabsorber (6, 7).

2. Wegwerfwindel für Erwachsene (1) nach Anspruch 1,
wobei die laminierte Schicht (3) ferner linke und rechte vierte Kerben (20, 21) umfasst, die sich in der Breitenrichtung der laminierten Schicht (3) nach innen in Richtung der zweiten Kerben (10, 11) im Grenzbereich zwischen dem mittleren Abschnitt (M) und dem hinteren Abschnitt (B) erstrecken.

3. Wegwerfwindel für Erwachsene (1) nach Anspruch 1,
ferner umfassend eine auslaufsichere Struktur (16a, 16b, 16c), die von einer Spitze bis zu einem Fußpunkt sowohl einer dem Träger zugewandten Fläche als auch einer gegenüberliegenden Fläche im linken und rechten Schenkelabsorber (6, 7) reicht.

## Revendications

1. Couche jetable pour adulte (1) comprenant :
un absorbeur (2) ;
une feuille stratifiée (3) dans laquelle une pluralité de feuilles sont stratifiées de manière à envelopper l'absorbeur (2) ; et
des unités froncées montantes gauche et droite (4, 5) destinées à s'étendre dans une direction longitudinale sur une surface faisant face à l'utilisateur (3a) de la feuille stratifiée (3),
dans laquelle la couche (1) est divisée en une section avant (F), une section médiane (M) et une section arrière (B) correspondant respectivement au bas-ventre, à l'entrejambe et aux fesses d'un utilisateur,
dans laquelle l'absorbeur (2) comprend des premières encoches gauche et droite (8, 9) destinées à s'étendre vers l'intérieur dans une direction de largeur de l'absorbeur (2) dans une région limite entre la section avant (F) et la section médiane (M) et comprend des deuxièmes encoches gauche et droite (10, 11) destinées à s'étendre vers l'intérieur dans la direction de largeur de l'absorbeur (2) dans une région limite entre la section médiane (M) et la section arrière (B),
dans laquelle la feuille stratifiée (3) comprend des troisièmes encoches gauche et droite (18, 19) destinées à s'étendre vers l'intérieur dans une direction de largeur de la feuille stratifiée (3) vers les premières encoches (8, 9) dans la région limite entre la section avant (F) et la partie médiane (M),
dans laquelle des absorbeurs pour cuisse gauche et droit (6, 7) qui sont utilisés en étant pliés de manière à faire face à l'intérieur des cuisses dans l'entrejambe de l'utilisateur sont formés par des parties latérales gauche et droite de l'absorbeur (2) dans la section médiane (M),
dans laquelle des corps élastiques (22a, 22b, 22c, 22d) sont agencés au niveau des racines des absorbeurs pour cuisse gauche et droit (6, 7) dans une région opposée à la surface faisant face à l'utilisateur (3a) dans la feuille stratifiée (3), et
dans laquelle des rainures évidées gauche et droite (23, 24) destinées à s'étendre dans la direction avant-arrière sont agencées plus près du centre de l'absorbeur (2) que les racines des absorbeurs pour cuisse gauche et droit (6,7).

2. Couche jetable pour adulte (1) selon la revendication 1,
dans laquelle la feuille stratifiée (3) comprend en outre des quatrièmes encoches gauche et droite (20, 21) destinées à s'étendre vers l'intérieur dans la direction de largeur de la feuille stratifiée (3) vers les deuxièmes encoches (10, 11) dans la région limite entre la section médiane (M) et la section arrière (B).

3. Couche jetable pour adulte (1) selon la revendication 1,
comprenant en outre une structure étanche (16a, 16b, 16c) qui recouvre depuis une pointe jusqu'à une racine à la fois d'une surface faisant face à l'utilisateur et une surface opposée à celle-ci dans les absorbeurs pour cuisse gauche et droit (6, 7).
